(19) Europäisches Patentamt
European Patent Office
Office européen des brevets

(11) **EP 2 427 562 B1**

(12) **EUROPEAN PATENT SPECIFICATION**

(45) Date of publication and mention
of the grant of the patent:
**09.10.2019 Bulletin 2019/41**

(51) Int Cl.:
*C12P 7/18* (2006.01)        *C12N 1/36* (2006.01)
*C12N 1/20* (2006.01)

(21) Application number: **10716352.9**

(22) Date of filing: **05.05.2010**

(86) International application number:
**PCT/EP2010/056078**

(87) International publication number:
**WO 2010/128070 (11.11.2010 Gazette 2010/45)**

(54) **Continuous culture for 1,3-propanediol production using a high glycerine concentration**

Kontinuierliche Kultur zur 1,3-Propandiol-Herstellung unter Verwendung einer hohen Glycerinkonzentration

Culture continue pour la production de 1,3-propanediol utilisant une concentration élevée en glycérine

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **05.05.2009 EP 09159401**
**05.05.2009 US 175564 P**

(43) Date of publication of application:
**14.03.2012 Bulletin 2012/11**

(73) Proprietor: **Metabolic Explorer**
**63360 Saint Beauzire (FR)**

(72) Inventors:
• **CHATEAU, Michel**
  **F-63200 Riom (FR)**
• **DUBOIS, Jean-Yves**
  **F-63360 Saint Beauzire (FR)**
• **SOUCAILLE, Philippe**
  **F-31450 Deyme (FR)**

(74) Representative: **Regimbeau**
**87 rue de Sèze**
**69477 Lyon Cedex 06 (FR)**

(56) References cited:
**EP-A1- 1 892 300    WO-A2-2009/108748**

• WITTLICH P: "Biotechnische Herstellung von 1,3-Propandiol aus Glycerin mit immobilisierten Zellen von Clostridium butyricum NRRL B-1024 und thermophilen Mikroorganismen", DISSERTATION, UNIVERSITÄT BRAUNSCHWEIG, NATURWISSENSCHAFTLICHE FAKULTÄT, [Online] 2001, pages I-130, XP002552274, Retrieved from the Internet: URL:http://rzbl04.biblio.etc.tu-bs.de:8080/docportal/servlets/MCRFileNodeServlet/DocPortal_derivate_00001238/Document.pdf> [retrieved on 2009-10-23]
• PETITDEMANGE E ET AL: "Fermentation of raw glycerol to 1,3-propanediol by new strains of Clostridium butyricum", JOURNAL OF INDUSTRIAL MICROBIOLOGY, vol. 15, no. 6, 1 January 1995 (1995-01-01), pages 498-502, XP008091022, SOCIETY FOR INDUSTRIAL MICROBIOLOGY ISSN: 0169-4146
• YING MU ET AL: "Microbial production of 1,3-propanediol by Klebsiella pneumoniae using crude glycerol from biodiesel preparations", BIOTECHNOLOGY LETTERS, vol. 28, no. 21, 10 August 2006 (2006-08-10), pages 1755-1759, XP019433293, KLUWER ACADEMIC PUBLISHERS, DO ISSN: 1573-6776
• CHENG ET AL: "Pilot-scale production of 1,3-propanediol using Klebsiella pneumoniae", PROCESS BIOCHEMISTRY, vol. 42, no. 4, 19 March 2007 (2007-03-19) , pages 740-744, XP005933491, ELSEVIER, NL ISSN: 1359-5113

EP 2 427 562 B1

**(Cont. next page)**

- GUANG YANG ET AL: "Fermentation of 1,3-propanediol by a lactate deficient mutant of Klebsiella oxytoca under microaerobic conditions", APPLIED MICROBIOLOGY AND BIOTECHNOLOGY, vol. 73, no. 5, 8 September 2006 (2006-09-08), pages 1017-1024, XP019472490, SPRINGER, BERLIN, DE ISSN: 1432-0614
- HIMMI EL HASSANE ET AL: "Nutrient requirements for glycerol conversion to 1,3-propanediol by Clostridium butyricum", BIORESOURCE TECHNOLOGY, vol. 67, no. 2, February 1999 (1999-02), pages 123-128, XP002552275, ISSN: 0960-8524
- DA SILVA G P ET AL: "Glycerol: A promising and abundant carbon source for industrial microbiology", BIOTECHNOLOGY ADVANCES, vol. 27, no. 1, 1 January 2009 (2009-01-01), pages 30-39, XP025781146, ELSEVIER PUBLISHING, BARKING, GB ISSN: 0734-9750 [retrieved on 2008-08-16]
- FERENCI THOMAS: "Bacterial physiology, regulation and mutational adaptation in a chemostat environment.", ADVANCES IN MICROBIAL PHYSIOLOGY 2008, vol. 53, 2008, pages 169-229, XP008113974, ISSN: 0065-2911
- WILLKE THOMAS ET AL: "Biotransformation of glycerol into 1,3-propanediol", EUROPEAN JOURNAL OF LIPID SCIENCE AND TECHNOLOGY, vol. 110, no. 9, Sp. Iss. SI, September 2008 (2008-09), pages 831-840, XP002552277, ISSN: 1438-7697
- HIRSCHMANN S ET AL: "Development of an integrated bioconversion process for the production of 1,3-propanediol from raw glycerol waters", LANDBAUFORSCHUNG, vol. 55, no. 4, 1 December 2005 (2005-12-01), pages 261-267, XP002552278,
- GONZALEZ-PAJUELO ET AL: "Metabolic engineering of Clostridium acetobutylicum for the industrial production of 1,3-propanediol from glycerol", METABOLIC ENGINEERING, vol. 7, no. 5-6, 1 September 2005 (2005-09-01), pages 329-336, XP005207140, ACADEMIC PRESS, US ISSN: 1096-7176 cited in the application

Remarks:

The file contains technical information submitted after the application was filed and not included in this specification

## Description

The present invention concerns a new method for the production of 1,3-propanediol comprising culturing a microorganism on a culture medium with high glycerine content. The invention also concerns a new microorganism, or strain of microorganism, adapted for the production of 1,3-propanediol from a medium comprising high glycerine content. The invention also concerns an "adapted microorganism" which glycerol metabolism is directed to 1,3-propanediol production, and which is allowed to grow in the presence of a high concentration of industrial glycerine. The disclosure also concerns a biosourced 1,3-propanediol obtained by the process of the invention. Finally the disclosure concerns the use of the above described biosourced 1,3-propanediol as extender chain in thermoplastic polyurethane, as monomers in polytrimethylene terephtalate and as a component in cosmetics formulations.

## Background of the invention

1,3-Propanediol (PDO) is one of the oldest known fermentation products. It was reliably identified as early as 1881 by August Freund, in a glycerol-fermenting mixed culture obviously containing *Clostridium pasteurianum* as the active organism. Quantitative analysis of the fermentation of different enterobacteria producing PDO (trimethylene glycol, propylene glycol) started at the microbiology school of Delft as early as 1928 and was successfully continued at Ames, Iowa in the 1940s. In the 1960s, interest shifted to the glycerol-attacking enzymes, in particular to the glycerol and diol dehydratases, as these enzymes were peculiar in requiring coenzyme B12. PDO-forming clostridia were first described in 1983 as part of a process to obtain a specialty product from glycerol-excreting algae (Nakas *et al.*, 1983). PDO is a typical product of glycerol fermentation and has not been found in anaerobic conversions of other organic substrates. Only very few organisms, all of them bacteria, are able to form it. They include enterobacteria of the genera *Klebsiella* (*K. pneumoniae*), *Enterobacter* (*E. agglomerans*) and *Citrobacter* (*C. freundii*), lactobacilli (*L. brevis* and *L. buchneri*) and clostridia of the C. *butyricum* and the C. *pasteurianum* group.

Analysis of the fermentation products shows that part of the glycerol is converted to the same products as in the sugar fermentation of this different species, namely acetic acid, 2,3-butanediol, butyric acid, lactic acid, ethanol and succinic acid. This conversion provides the necessary energy for growth but, for many of the products, reducing equivalents are also released, which are used in a reductive conversion of glycerol to PDO. Butyrate formation, which lowers the PDO yield in clostridia, is somewhat comparable to ethanol formation in *Klebsiella*, but it seems to be more dependent on growth rate. Butyrate decreases rapidly with the dilution rate, even in the absence of substrate excess. In any case, changes in the acetate/butyrate ratio do not have such a great impact on the PDO yield.

PDO, as a bi-functional organic compound, could potentially be used for many synthesis reactions, in particular as a monomer for polycondensations to produce polyesters, polyethers and polyurethanes. PDO can be produced by different chemicals routes but they generate waste streams containing extremely polluting substances and the cost of production is then high and chemically produced PDO could not compete with the petrochemically available, diols like 1,2-ethanediol, 1,2-propanediol, and 1,4-butanediol. Therefore, in the past PDO has only found niche applications of negligible market volume. It is the reason why in 1995 Dupont started a research program for the biological conversion of glucose to PDO. Although this process is environmentally friendly it has the disadvantage to i) use vitamin B12 a very expensive cofactor and ii) be a discontinuous process due to the instability of the producing strain. Due to the availability of large amount of glycerol issued from bio-diesel industry, a continuous process, vitamin B12 free with a higher carbon yield would be advantageous.

It is known in the art that PDO can be produced from glycerine, an unwanted by product of the biodiesel production which contains roughly 80-85% of glycerol mixed with salts and water.

*C. butyricum* was previously described as been able to grow and produce PDO from industrial glycerol in batch and two-stage continuous fermentation (Papanikolaou *et al.*, 2000). However, at the highest glycerol concentration, the maximal PDO titre obtained was 48.1 g/L at a dilution rate of $0.02h^{-1}$, meaning a productivity of 0.9 g/L/H. The cultures were conducted with a maximum glycerol concentration in the fed medium of 90g/L and in the presence of yeast extract, a costly compound containing organic nitrogen that is known by the man skilled in the art to help in increasing bacterial biomass production.

WO2006/128381 discloses the use of this glycerine for the production of PDO with batch and Fed-Batch cultures using natural PDO producing organisms such as *Klebsiella pneumoniae, C. butyricum* or *C. pasteuricum..Furthermore*, the medium used in WO2006/128381 also contains yeast extract. As described in this patent application, the maximal productivity reached is comprised between 0.8 and $1.1g.l.h^{-1}$.

The performance of a C. *acetobutylicum* strain modified to contain the vitamin B12-independent glycerol-dehydratase and the PDO-dehydrogenase from C. *butyricum,* called *C. acetobutylicum* DG1 pSPD5 has been described in Gonzalez-Pajuelo *et al.*, 2005. This strain originally grows and produces PDO with a fed medium containing up to $120g.l^{-1}$ of pure glycerol. In addition, analyses with a fed medium of containing a maximum $60g.l^{-1}$ of pure or industrial glycerol did not identify any differences. When comparing *C. butyricum* to *C. acetobutylicum* DG1 pSPD5 a global similar behaviour was observed by the authors Gonzalez-Pajuelo *et al.*, 2006.

However, the same *C. acetobutylicum* DG1 pSPD5 strain tested with 105g.l$^{-1}$ of industrial glycerol in a synthetic fed medium without organic nitrogen, could not give the same performances as with the same concentration of pure glycerol (see example 1).

The present invention provides means for the production of 1,3-propandeiol with high concentration of industrial glycerine and where a higher PDO titre and productivity can be reached.

**Brief description of the invention**

The present invention concerns a method for the production of 1,3-propanediol in a continuous fermentation process of glycerine, comprising culturing a strain of *Clostridium* genetically modified to have its glycerol metabolism directed to 1,3-propanediol production on a culture medium, and recovering the 1,3-propanediol, characterized in that the feed medium comprises a high concentration of industrial glycerine, said industrial glycerine being a by-product from biodiesel production and comprising of more than 70% of glycerol, said glycerol being present at a concentration comprised between 90 and 120g/l, and the dilution rate is comprised between 0.005 and 0.1h$^{-1}$, and wherein the strain of *Clostridium* is previously adapted to grow in the presence of a high concentration of industrial glycerine by a continuous culture at a dilution rate comprised between 0.005 and 0.1h$^{-1}$ in the presence of a high concentration of industrial glycerine in the feed medium, said industrial glycerine comprising of more than 70% of glycerol, said glycerol being present at a concentration comprised between 90 and 120g/l, and wherein said culture medium does not contain any organic nitrogen source.

In preferred embodiments, the glycerol is present at a concentration of about 105g/L.

The culture medium is preferably a synthetic medium, and in particular without yeast extract.

The strain of *Clostridium* genetically modified to have its glycerol metabolism directed to 1,3-propanediol production is preferably *Clostridium acetobutylicum.*

In a preferred embodiment, the glycerol dehydratase activity in the strain of *Clostridium* genetically modified is independent of the presence of coenzyme B12 or one of its precursors and is derived from *Clostridium butyricum.*

Preferably, genetically modified strain of *Clostridium* is previously adapted to grow on the culture medium having a high concentration of industrial glycerine by culturing the microorganism on a culture medium comprising a high concentration of industrial glycerine at a low dilution rate comprised between 0.005 and 0.1h$^{-1}$ and selecting the adapted microorganism.

Eventually, the 1,3-propanediol is further purified.

The present invention also concerns a method for the modification of a microorganism of the genus *Clostridium* into a microorganism adapted to grow in the presence of a high concentration of industrial glycerine, said industrial glycerine being a by-product from biodiesel production and comprising more than 70% of glycerol, said method comprising continuously culturing the microorganism on a culture medium not containing any organic nitrogen source, the feed medium comprising a high concentration of industrial glycerine, the glycerol being present at a concentration comprised between 90 and 120 g/l, and being fed at a low dilution rate comprised between 0.005 and 0.1h$^{-1}$ and over a period ranging from 24 hours to 10 days, and selecting the adapted microorganism able to grow on the culture medium having a high concentration of industrial glycerine.

The microorganism is cultured at low dilution rate over a period ranging from 24 hours to 10 days, preferably more than 2 days, more preferably about 8 days.

The dilution rate is comprised between 0.005 and 0.1 h$^{-1}$, preferably between 0.005 and 0.02 h$^{-1}$. The dilution rate can be changed during the adaptation method, eventually with a first step comprised between 0.005 and 0.02 h$^{-1}$ and a second step where the dilution rate is increased up to 0.1 h$^{-1}$, preferably 0.06 h$^{-1}$.

The present invention also concerns an adapted microorganism, a producing microorganism of the genus *Clostridium* genetically modified to have its glycerol metabolism directed to 1,3-propanediol production adapted to grow in the presence of a high concentration of industrial glycerine obtainable by the method as disclosed above and below.

The disclosure also concerns a biosourced 1,3-propanediol obtained by the method of the invention.The disclosure also concerns a biosourced 1,3-propanediol characterized by a combination of $^{13}$C and $^{18}$O isotopic values selected among $\delta^{13}$C lower than -34 ‰ and $\delta^{18}$O between 21,9 ‰ and 0,5 ‰, preferably $\delta^{13}$C is lower than -35 ‰ and $\delta^{18}$O is between 21,9 ‰ and 0,5 ‰, more preferably $\delta^{13}$C is between -35,05 ‰ and -36,09 ‰ and $\delta^{18}$O is between 21,9 ‰ and 17,34 ‰.

The biosourced 1,3-propanediol is characterized by a $\delta^{13}$C/$\delta^{18}$O isotopic ratio value comprised between -2 and 0, preferably between -1 and -0,2 and more preferably between -0,65 and -0,4.

The present disclosure also concerns the use of biosourced 1,3-propanediol obtained by the method of the invention, or as defined above, as an extender chain in thermoplastic polyurethane, as monomers in polytrimethylene terephtalate or as a component in cosmetic formulations.

## Detailed description of the invention

The invention is related to a method for the production of 1,3-propanediol in a continuous fermentation process of glycerine, comprising culturing a strain of *Clostridium* genetically modified to have its glycerol metabolism directed to 1,3-propanediol production on a culture medium, and recovering the 1,3-propanediol, characterized in that the feed medium comprises a high concentration of industrial glycerine, said industrial glycerine being a by-product from biodiesel production and comprising of more than 70% of glycerol, said glycerol being present at a concentration comprised between 90 and 120g/l, and the dilution rate is comprised between 0.005 and 0.1h$^{-1}$, and wherein the strain of *Clostridium* is previously adapted to grow in the presence of a high concentration of industrial glycerine by a continuous culture at a dilution rate comprised between 0.005 and 0.1h$^{-1}$, said industrial glycerine comprising of more than 70% of glycerol, said glycerol being present at a concentration comprised between 90 and 120g/l, and wherein said culture medium does not contain any organic nitrogen source.

The strain of *Clostridium* is preferably *Clostridium acetobutylicum* and *Clostridium butyricum.*

The term *"Clostridium"* refer to all kind of bacteria belonging to this family or genus.

The term "producing strain of *Clostridium"* or "producing strain of *Clostridium"* means a microorganism or a strain of microorganism wherein the glycerol metabolism of the microorganism is directed to 1,3-propanediol production.

A "microorganism being adapted" means a microorganism being modified to be able to grow on high concentrations of industrial glycerine.

An "appropriate culture medium" or a "culture medium" refers to a culture medium optimized for the growth and the diol-production of the producing strain.

The terms "high glycerine content" or "high concentration of glycerine" means the culture medium comprises glycerol at a concentration comprised between 90 and 120 g/L, preferably about 105g/L.

"Industrial glycerine" means a glycerine product obtained from an industrial process without substantial purification. Industrial glycerine can also be designated as "raw glycerine", "raw glycerol" or "industrial glycerol". Industrial glycerine contains more than about 70% of glycerol, preferably more than about 80 %, less than 15% of water and impurities such as mineral salts and fatty acids. The concentration of mineral salts is less than 10%, preferably less than 5%. The concentration of fatty acids is less than 20%, preferably less than 5%. Fatty acids most represented in the industrial glycerine are palmitic acid, stearic acid, oleic acid, linolenic acid, linoleic acid and arachidic acid.

Industrial processes from which industrial glycerine is obtained are, inter alia, manufacturing methods where fats and oils, particularly fats and oils of plant origin, are processed into industrial products such as detergent or lubricants. In such manufacturing methods, industrial glycerine is considered as a by-product.

In a particular embodiment, the industrial glycerine comprises known impurities of glycerine obtained from biodiesel production, comprising about 80 to 85% of glycerol with salts, water and some other organic compounds such as fatty acids. Industrial glycerine obtained from biodiesel production has not been subjected to further purification steps.

The term "synthetic medium" means a culture medium comprising a chemically defined substrate upon which organisms are grown.

In the culture medium of the present invention, glycerol is advantageously the single source of carbon. This culture medium does not contain any organic nitrogen source. Nitrogen is a naturally occurring element that is essential for growth and reproduction in both plants and animals. It is found in amino acids and in many other organic and inorganic compounds. "Organic nitrogen" means, according to the invention, a nitrogen comprising organic compound obtained from living organisms. Usual sources of organic nitrogen for bacterial culture comprise yeast extract.

In a preferred embodiment, the *Clostridium* strain is *Clostridium acetobutylicum.*

The phrase "a strain of *Clostridium* genetically modified " means that the strain has been transformed in the aim to change its genetic characteristics. Endogenous genes can be attenuated, deleted, or over-expressed. Exogenous genes can be introduced, carried by a plasmid, or integrated into the genome of the strain, to be expressed into the cell.

The term "plasmid" or "vector" as used herein refers to an extra chromosomal element often carrying genes which are not part of the central metabolism of the cell, and usually in the form of circular double-stranded DNA molecules.

In another embodiment of the invention, the method is characterized in that the genetically modified strain of *Clostridium* has a glycerol dehydratase activity that is independent of the presence of coenzyme B12 or one of its precursors, and that is derived from *Clostridium butyricum.*

In particular, the genetically modified strain of *Clostridium* presents an increased flux of 1,3-propanediol production by introducing extra copies of the 1,3-propanediol operon from C. *butyricum,* (coding for enzymes involved in the vitamin B12-independent 1,3-propanediol pathway) either over-expressed by a plasmid or integrated into the chromosome of the microorganism. For example the pSPD5 plasmid can be used for an over-expression of the 1,3-propanediol operon.

Method for directing the glycerol metabolism towards production of 1,3-propanediol are known in the art (see for instance WO2006/128381, Gonzalez-Pajuelo & al. 2006).

In the present invention, the producing strain of *Clostridium* has been previously adapted to grow in the presence of a high concentration of industrial glycerine. Said "adaptation" of the producing strain of *Clostridium* is obtained by continuously culturing the strain of *Clostridium* at a dilution rate comprised between 0.005 and 0.1h$^{-1}$ in the presence of a high concentration of industrial glycerine in the feed medium, said industrial glycerine comprising of more than 70% of glycerol, said glycerol being present at a concentration comprised between 90 and 120g/l, and selecting the adapted microorganism able to grow on the culture medium having high concentration of industrial glycerine.

The present invention is also related to a method for the modification of a microorganism of the genus *Clostridium* into a microorganism adapted to grow in the presence of a high concentration of industrial glycerine.

Several "adaptation processes" may be chosen by the person skilled in the art to transform the producing microorganism into a producing microorganism allowed to grow in the presence of a high concentration of industrial glycerine.

According to the present invention, the modification of a microorganism of the genus *Clostridium* into a microorganism adapted to grow in the presence of a high concentration of industrial glycerine being a by-product from biodiesel production and comprising more than 70% of glycerol, comprises continuously culturing the microorganism on a culture medium not containing any organic nitrogen source, the feed medium comprising a high concentration of industrial glycerine the glycerol being present at a concentration comprised between 90 and 120 g/l, and being fed at a low dilution rate comprised between 0.005 and 0.1h$^{-1}$ and over a period ranging from 24 hours to 10 days, and selecting the adapted microorganism able to grow on the culture medium having a high concentration of industrial glycerine.

The microorganism is cultured at a low dilution rate over a period ranging from 24 hours to 10 days, preferably more than 2 days, more preferably about 8 days.

The dilution rate is comprised between 0,005 and 0,1 h$^{-1}$, preferably between 0,005 and 0,02 h$^{-1}$. The dilution rate can be changed during the adaptation method, eventually with a first step comprised between 0,005 and 0,02 h$^{-1}$ and a second step where the dilution rate is increased up to 0,1 h$^{-1}$, more preferably 0,06 h$^{-1}$. When the dilution rate is modified during the adaptation method, dilution rate between 0.005 and 0.02 h$^{-1}$ are called "low dilution rate" while dilution rate between 0.02 and 0.1 h$^{-1}$ are common dilution rate.

The invention is also related to a microorganism of the genus *Clostridium* genetically modified to have its glycerol metabolism directed to 1,3-propanediol production adapted to grow in the presence of a high concentration of industrial glycerine and in a culture medium which does not contain any organic nitrogen source susceptible to be obtained by the method as described above, said industrial glycerine being a by-product from biodiesel production and comprising more than 70% of glycerol, and in the feed medium the glycerol of a continuous culture being present at a concentration comprised between 90 and 120g/l.

The microorganism adapted with the method of the present invention is preferably a producing microorganism being further adapted to grow in the presence of a high concentration of industrial glycerine.

Said adapted microorganism can also be a microorganism first adapted to grow in the presence of a high concentration of industrial glycerine further modified to have its glycerol metabolism directed to 1,3-propanediol production.

The "adapted microorganism" according to the invention is a producing microorganism adapted to grow in the presence of a high concentration of industrial glycerine and has two essential characteristics:

- its glycerol metabolism is directed to 1,3-propanediol production, and
- it is allowed to grow in the presence of a high concentration of industrial glycerine as defined herein.

In a specific embodiment of the invention, the *C. acetobutylicum* DG1 pSPD5 strain is cultivated in continuous culture using a feed medium containing 105g.l$^{-1}$ of raw glycerol from the biodiesel production, at a low dilution rate comprised between 0.005 and 0.02h$^{-1}$, preferably 0.02h$^{-1}$. Over a period of maximum 10 days, preferably between 5 and 8 days, the strain is adapted to the high glycerine concentration present in the fed medium, and the dilution rate can be increased up to 0.1 h$^{-1}$, preferably up to 0.06 h$^{-1}$ (see example 2).

The gradual increase of the dilution rate can be done between the end of the batch phase and 10 days, preferentially after 5 days, between 5 and 8 days, about 7 days, resulting in an improved productivity of the continuous culture (see example 3).

Advantageously, after the adaptation phase, the glycerol concentration in the fed medium can be increased up to 120g.l$^{-1}$. However, direct attempt to adapt the strain to with 120g.l$^{-1}$ of industrial glycerol is not possible, even at a dilution rate of 0.02h$^{-1}$ as for a glycerol concentration of 105g.l$^{-1}$, again demonstrating that the strain is not able to grow in the presence of a high glycerine concentration in the fed medium without prior adaptation.

The method of the invention by the use of a genetically modified microorganism and of medium without additional organic nitrogen source leads to production of 1,3-propanediol with a yield comprised between 0,4 and 0.6 g.g$^{-1}$ and a productivity comprised between 1.8 and 3.5 g.l$^{-1}$.h$^{-1}$ for a dilution rate comprised between 0.05 and 0.6g.h$^{-1}$. Preferably the yield is comprised between 0.5 and 0.56 g.g$^{-1}$ and the productivity between 2 and 2.9 g.l$^{-1}$.h$^{-1}$.

In a specific aspect of the invention, the produced 1,3-propanediol is furthermore purified.

Continuous fermentation processes are known to the person skilled in the art.

The fermentation process is generally conducted in reactors with an inorganic culture medium of known defined composition adapted to the bacteria used, containing at least glycerine, a by-product from biodiesel production containing glycerol, and if necessary a co-substrate for the production of the metabolite.

This method of the invention is realized in a continuous process. The man skilled in the art knows how to manage each of these experimental conditions, and to define the culture conditions for the microorganisms according to the invention. In particular clostridia are fermented at a temperature between 20°C and 60°C, preferentially between 25°C and 40°C for *C. acetobutilicum.*

Methods for recovering and eventually purifying 1,3-propanediol from a fermentation medium are known to the skilled person. 1,3-propanediol may be isolated by distillation. In most embodiment, 1,3-propanediol is distilled from the fermentation medium with a by-product, such as acetate, and then further purified by known methods.

The present disclosure also concerns biosourced 1,3-propanediol obtained according to the method described above.

The present disclosure also concerns biosourced 1,3-propanediol characterized by its isotope ratios. Analysis of isotope ratios of hydrogen (D/H) and carbon ($^{13}$C/$^{12}$C) provides authenticity indicators for specific products such as honey (Grenier-Loustalot et al., 2006) or wine (Guillou et al, 2001). The $^{13}$C/$^{12}$C isotope ratio provides indications for source discrimination and reflects the biosynthetic metabolic pathway of the specific product and the used raw material. Indeed difference could be made between C3 plants using Calvin-Benson photosynthetic cycle and C4 plants using Hatch-Slack photosynthetic cycle. The patent application WO 01/11070 describes a $^{13}$C/$^{12}$C isotope ratio of biosourced 1,3-propanediol produced from glucose between -10,9 and -15,4; a $^{13}$C/$^{12}$C isotope ratio of biosourced 1,3-propanediol produced from glycerol between - 22,41 and -22,60 while $^{13}$C/$^{12}$C isotope ratio of chemically produced 1,3-propanediol is comprised between -17,95 and -18,33.

According to the present disclosure, 1,3-propanediol D/H ratio (noted $\delta$D), $^{13}$C/$^{12}$C ratio (noted $\delta^{13}$C), $^{18}$O/$^{16}$O ratio (noted $\delta^{18}$O) were determined by mass spectrometry after combustion. The isotope $^{13}$C/$^{12}$C ratio was calculated as $\delta$ per mill (‰) with reference to the international standard (PDB = Pee Dee Belemite). The isotope $^{18}$O/$^{16}$O ratio was calculated as $\delta$ per mill (‰) with reference to the international standard Mean Ocean Water (SMOW). The isotope D/H ratio was calculated as ppm compare to the international standard Mean Ocean Water (SMOW). Even though D/H ratio and $^{13}$C/$^{12}$C ratios are well known for the characterisation of a given product, $^{18}$O/$^{16}$O ratio is mainly used for water analysis as a paleoclimatic indicator.

Comparison between different types of 1,3-propanediol shows that $\delta$D is not discriminatory. $\delta$D for 1,3-propanediol biosourced from glycerol (object of the invention) is between 147.38 ppm and 145.84 ppm whereas is 145 ppm for 1,3-propanediol biosourced from glucose and between 150.19 ppm and 139.37 ppm for chemical 1,3-propanediol.

The disclosure concerns the biosourced 1,3-propanediol susceptible to be obtained according to the method described above, characterized by $\delta^{13}$C isotopic values lower than -34 ‰, preferably lower than -35 ‰ and more preferably comprised between -35.05 ‰ and -36.09 ‰. The disclosure also concerns the biosourced 1,3-propanediol susceptible to be obtained according to the method described above, characterized by $\delta^{18}$O isotopic values comprised between 21.5 ‰ and 0.5 ‰, preferably between 21.9 ‰ and 15 ‰ and more preferably comprised between 21.9 ‰ and 17.34 ‰.

The disclosure also concerns a biosourced 1,3-propanediol characterized by one of the following characteristics or a combination thereof:

- $^{13}$C and $^{18}$O isotopic values selected among $\delta^{13}$C lower than -34 ‰ and $\delta^{18}$O between 21.9 ‰ and 0.5 ‰,
- preferably $\delta^{13}$C is lower than -35 ‰ and $\delta^{18}$O is between 21.9 ‰ and 0.5 ‰,
- more preferably $\delta^{13}$C is between -35.05 ‰ and -36.09 ‰ and $\delta^{18}$O is between 21.9 ‰ and 17.34 ‰ (See example 4).

The biosourced 1,3-propanediol is characterized by a $^{18}$O/$^{13}$C isotopic ratio value comprised between -2 and 0; preferably between -1 and -0.2 and more preferably between -0.65 and -0.4.

Preferably, biosourced 1,3-propanediol characterized by the previous isotope ratios is obtained by fermentation based on glycerol as raw material. More preferably, biosourced 1,3-propanediol characterized by the previous isotope ratios is obtained from the method of the invention for the production of 1,3-propanediol.

The present disclosure also concerns the use of biosourced 1,3-propanediol obtained by the method of the invention, or as defined above, as extender chain in thermoplastic polyurethanes, as monomers in polytrimethylene terephtalate or as component in cosmetic formulations. Biosourced 1,3-propanediol may be used in all known applications of chemical 1,3-propanediol. The man skilled in the art knows how to obtain these final products from 1,3-propanediol.

The present disclosure concerns methods of preparation of thermoplastic polyurethanes using as extender chain a biosourced 1,3-propanediol according to the invention. It also concerns methods of preparation of cosmetic compositions containing biosourced 1,3-propanediol according to the invention. It also concerns methods of synthesis of polytrimethylene terephtalate using biosourced 1,3-propanediol according to the invention as monomer.

**Drawings**

Figure 1: PDO $\delta^{13}C$ and $\delta^{18}O$ in ‰ of the international standard: PDO1: PDO produced according to the described process; PDO2: PDO produced from glucose as a substrate; PDO3: PDO produced by chemical process.
Figure 2: $\delta^{18}O$ / $\delta^{13}C$ ratio of PDO: PDO1: PDO produced according to the described process; PDO2: PDO produced from glucose as a substrate; PDO3: PDO produced by chemical process.

**Examples**

**Example 1**

The synthetic media used for *Clostridium* batch cultivations contained per litre of de-ionized water: glycerol, 30g; $KH_2PO_4$, 0.5; $K_2HPO_4$, 0.5g; $MgSO_4$, $7H_2O$, 0.2g; $CoCl_2$ $6H_2O$, 0.01g, $H_2SO_4$, 0.1ml; $NH_4Cl$, 1.5g, biotin, 0.16mg; p-amino benzoic acid, 32mg and $FeSO_4$, $7H_2O$, 0.028g. The pH of the medium was adjusted to 6.3 with $NH_4OH$ 3N. For batch cultivation commercial glycerol purchased from Sigma (purity 99.5%) was used. The feed medium for continuous cultures contained par litre of tap water: raw glycerol, 105g; $KH_2PO_4$, 0.5; $K_2HPO_4$, 0.5g; $MgSO_4$, $7H_2O$, 0.2g; $CoCl_2$ $6H_2O$, 0.026g; $NH_4Cl$, 1.5g, biotin, 0.16mg; p-amino benzoic acid, 32mg; $FeSO_4$, $7H_2O$, 0.04g, antifoam, 0,05ml; $ZnSO_4$, $7H_2O$, 8mg; $CuCl_2$, $2H_2O$, 4mg; $MnSO_4$, $H_2O$, 40mg, $H_3BO_3$, 2mg; $Na_2MoO_4$, $2H_2O$, 0.8mg. Medium pH was not adjusted in this case. Raw glycerol resulting from the trans-esterification process of biodiesel production was supplied by SAIPOL (Le Meriot, France) and contained 83%glycerol (w/w).

Continuous cultures were performed in a 2L bioreactor Tryton (Pierre Guerin, France) with a working volume of 1000ml. The culture volume was kept constant at 1000ml by automatic regulation of the culture level. Cultures were stirred at 200 RPM, at a temperature of 35°C and pH was maintained at 6.5 by automatic addition of $NH_4OH$ 3N. The Oxido-Reductive Power of the culture medium expressed in mV, was controlled and recorded during the experiment. To create anaerobic conditions, the sterilized medium in the vessel was flushed with sterile O2-free nitrogen for one hour at 60°C and flushed again until 35°C was reached. The bioreactor gas outlet was protected form the oxygen by a pyrogallol arrangement (Vasconcelos *et al.*, 1994). After sterilisation the feed medium was also flushed with sterile O2-free nitrogen until room temperature was attained and maintained under nitrogen pressure at 200mbar to avoid O2 entry.

Cell concentration was measured turbidimetrically at 620nm and correlated with cell dry weight evaluated directly. Glycerol, 1,3-propanediol, ethanol, butanol, acetic and butyric acids and others trace levels acids concentrations were measured by HPLC analysis. Separation was performed on a Biorad Aminex HPX-87H column and detection was achieved by refractive index. Operating conditions were as follows: mobile phase sulphuric acid 0.5mM; flow rate 0.5ml/min, temperature, 25°C.

A growing culture in 100ml flasks on synthetic medium (the same as the batch culture medium described above but with the addition of acetic acid, $2.2g.l^{-1}$ and MOPS, $23.03g.l^{-1}$) taken at the end of exponential growth phase was used as inoculums (5% v/v). The cultures were first grown batch-wise. At the early exponential growth phase a pulse of commercial glycerol was added: the pulse is defined by an addition of synthetic medium (the same as described for batch culture) with commercial glycerol $120g.l^{-1}$ at a flow rate of $50ml.h^{-1}$ during 3 hours (*i.e.* an addition of 18g of glycerol). Then the growth continued batch-wise and before the end of the exponential growth phase the continuous feeding started with a dilution rate of $0.06h^{-1}$ and a feed medium containing $105g.l^{-1}$ of raw glycerol.

**Table 1:** Continuous culture of *C. acetobutylicum* DG1 (pSPD5) on raw glycerol at 105 g.l$^{-1}$ (D = 0.06h-1, pH 6.5 ant T°C = 35°C). The given values represent an average of 7 points in steady state condition obtained after 3 volume changes.

|  | Average and standard deviation |
| --- | --- |
| Feed glycerol (g.l$^{-1}$) | 106.1 +/-0.00 |
| 1,3-propanediol (g.l$^{-1}$) | 32.68 +/- 1.16 |
| $Y_{1,3\text{-}PDO}$ (g-g$^{-1}$) | 0.50 +/- 0.02 |
| $Q_{1,3PDO}$ (g.l$^{-1}$.h$^{-1}$) | 2.00 +/- 0.09 |
| | |
| Dilution rate (h$^{-1}$) | 0.061 +/-0.003 |
| Residual glycerol (g.l$^{-1}$) | 36.38 +/- 1.88 |
| Biomass (g.l$^{-1}$) | 1.83 +/-0.09 |
| Acetic acid (g.l$^{-1}$) | 0.99 +/- 0.07 |

(continued)

|  | Average and standard deviation |
|---|---|
| Butyric acid (g.l$^{-1}$) | 7.65 +/- 0.44 |
| Carbon recovery (%) | 96.9 +/- 2.5 |

$Y_{1,3-PDO}$ : PDO yield (g/g of glycerol consumed)
$Q_{1,3PDO}$ : PDO volumetric productivity
For the carbon recovery calculation, carbon dioxide concentration was estimated from end-products concentrations.

## Example 2

The synthetic media used were the same as described in example 1. Pre-culture, Inoculation and batch-wise growth were performed in the same conditions as described above.

The continuous feeding started with a dilution rate of 0.02h$^{-1}$ and a feed medium containing 105g.l$^{-1}$ of raw glycerol. After few days in these conditions (*i.e.* 8-10 days after inoculation of the bioreactor corresponding to 3 volumes changes at least) the dilution rate was increased from 0.02h$^{-1}$ to 0.05h$^{-1}$ according to the following scheme: i) increase of 0.01h$^{-1}$ unit in 48 hours and ii) resting step of 24-48 hours, repeated 3 times. Stability of the culture was followed by products analysis using the HPLC protocol previously described. Notably, we waited for residual glycerol to be as low as possible to make a final increased of the dilution rate to 0.06h$^{-1}$ in 48 hours.

Table 2: Continuous culture of *C. acetobutylicum* DG1 (pSPD5) on raw glycerol at 105 g.l$^{-1}$ (D = 0.05h-1 and 0.06h-1, pH 6.5 ant T°C = 35°C). The given values represent an average of 3 or 4 points in steady state condition.

|  | Average and standard deviation for D = 0,05h$^{-1}$ | Average and standard deviation for D = 0,06h$^{-1}$ |
|---|---|---|
| Feed glycerol (g.l$^{-1}$) | 108.36 +/- 0.00 | 110.01 +/- 0.00 |
| 1,3-propanediol (g.l$^{-1}$) | 51.92 +/- 0.29 | 50.95 +/- 0.52 |
| $Y_{1,3-PDO}$ (g-g$^{-1}$) | 0.53 +/- 0.00 | 0.51 +/- 0.01 |
| $Q_{1,3PDO}$ (g.l$^{-1}$.h$^{-1}$) | 2.68 +/- 0.04 | 2.87 +/- 0.02 |
| Dilution rate (h$^{-1}$) | 0.052 +/- 0.001 | 0.056 +/- 0.000 |
| Residual glycerol (g.l$^{-1}$) | 3.66 +/- 0.40 | 4.51 +/- 0.33 |
| Biomass (g.l$^{-1}$) | 1.94 +/- 0.07 | DO |
| Acetic acid (g.l$^{-1}$) | 3.27 +/- 0.11 | 2.67 +/- 0.06 |
| Butyric acid (g.l$^{-1}$) | 10.75 +/- 0.44 | 10.97 +/- 0.21 |
| Carbon recovery (%) | 97.4 +/- 1.4 | 95.1 +/- 1.2 |

$Y_{1,3-PDO}$ : PDO yield (g/g of glycerol consumed)
$Q_{1,3PDO}$ : PDO volumetric productivity
For the carbon recovery calculation, carbon dioxide concentration was estimated from end-products concentrations.

Another culture performed in the same conditions led to the same results.

## Example 3

The synthetic media used were the same as described in example 1, except that the feed medium contained 120g.l$^{-1}$ of raw glycerol. Pre-culture, Inoculation and batch-wise growth were performed in the same conditions as described above.

A) The continuous feeding started with a dilution rate of 0.02h$^{-1}$ and a feed medium containing directly 120g.l$^{-1}$ of raw glycerol.

Table 3: Continuous culture of *C. acetobutylicum* DG1 (pSPD5) on raw glycerol at 120 g.l$^{-1}$ (D = 0.02h$^{-1}$, pH 6.5 ant T°C = 35°C). The given values represent an average of 7 points in steady state condition obtained after 3 volume changes.

|  | Average and standard deviation |
|---|---|
| Feed glycerol (g.l$^{-1}$) | 123.67 +/- 0.00 |

(continued)

|  | Average and standard deviation |
|---|---|
| 1,3-propanediol (g.l$^{-1}$) | 43.13 +/- 1.47 |
| $Y_{1,3-PDO}$ (g-g$^{-1}$) | 0.50 +/- 0.01 |
| $Q_{1,3PDO}$ (g.l$^{-1}$.h$^{-1}$) | 1.01 +/- 0.05 |
| Dilution rate (h$^{-1}$) | 0.023 +/- 0.001 |
| Residual glycerol (g.l$^{-1}$) | 30.87 +/- 2.62 |
| Biomass (g.l$^{-1}$) | 1.19 +/- 0.09 |
| Acetic acid (g.l$^{-1}$) | 2.36 +/- 0.18 |
| Butyric acid (g.l$^{-1}$) | 9.32 +/- 0.45 |
| Carbon recovery (%) | 94.2 +/- 1.4 |

$Y_{1,3-PDO}$ : PDO yield (g/g of glycerol consumed)
$Q_{1,3PDO}$ : PDO volumetric productivity
For the carbon recovery calculation, carbon dioxide concentration was estimated from end-products concentrations.

**B)** The continuous feeding started with a dilution rate of 0.02h$^{-1}$ and a feed medium containing first 105g.l$^{-1}$ of raw glycerol. After few days in these conditions (corresponding to 3 volumes changes at least), the dilution rate was increased from 0.02h$^{-1}$ to 0.06h$^{-1}$ according to the scheme described previously. Then the feeding was switched to a dilution rate of 0.05h$^{-1}$ and a feed medium containing 120g.l$^{-1}$ of raw glycerol.

**Table 4:** Continuous culture of *C. acetobutylicum* DG1 (pSPD5) on raw glycerol at 120 g.l$^{-1}$ (D = 0.05h$^{-1}$, pH 6.5 ant T°C = 35°C) after feeding at 105g.l$^{-1}$ raw glycerol at D = 0.06h$^{-1}$. The given values represent an average of 3 points obtained after at least 20 volume changes.

|  | Average and standard deviation |
|---|---|
| Feed glycerol (g.l$^{-1}$) | 123.68 +/- 0.00 |
| 1,3-propanediol (g.l$^{-1}$) | 53.53 +/- 0.59 |
| $Y_{1,3-PDO}$ (g-g$^{-1}$) | 0.53 +/- 0.02 |
| $Q_{1,3PDO}$ (g.l$^{-1}$.h$^{-1}$) | 2.86 +/- 0.02 |
| Dilution rate (h$^{-1}$) | 0.053 +/- 0.000 |
| Residual glycerol (g.l$^{-1}$) | 15.58 +/- 3.56 |
| Biomass (g.l$^{-1}$) | 1.14 +/- 0.18 |
| Acetic acid (g.l$^{-1}$) | 4.30 +/- 0.10 |
| Butyric acid (g.l$^{-1}$) | 9.29 +/- 0.17 |
| Carbon recovery (%) | 94.8 +/- 2.3 |

$Y_{1,3-PDO}$ : PDO yield (g/g of glycerol consumed)
$Q_{1,3PDO}$ : PDO volumetric productivity
For the carbon recovery calculation, carbon dioxide concentration was estimated from end-products concentrations.

**Exemple 4**

**Methods for calculation of the $\delta^{13}$C and $\delta^{18}$O values**

Levels of $^{13}$C and $^{18}$O are by convention expressed as relative values. Normally they are expressed in the form of a percentage ($\delta$) when compared to two internationals references.
For the $^{13}$C, the reference is the "Vienna.PD belemnite" which is a fossil carbonate from which the $^{13}$C value is known.
The formula for the calculation of the $\delta$ is as follow:

$$\delta^{13}C(‰) = \frac{(^{13}C/^{12}C)_{spl} - (^{13}C/^{12}C)_{ref}}{(^{13}C/^{12}C)_{ref}} \times 1000$$

For the $^{18}O$ the reference is the "Standard Mean Ocean Water" (SMOW) with a known $^{18}O$ value. The formula is identical to the $^{13}C$ one, with the $^{18}O$ reference value.

Used materials and sample preparation

$^{13}C$

The $^{13}C/^{12}C$ isotopic ratio of PDO was calculated based on the carbon dioxide specimen measured experimentally. For this purpose the samples were burned in an elemental analyzer and the carbon dioxide obtained was injected into a mass spectrometer (Finnigan MAT DELTA) coupled to an elemental analyzer (CARLO ERBA NA 2100) for the determination of the isotopic ratio. In this way the 44, 45 and 46 masses of carbon dioxide were separated and quantified. The $^{13}C/^{12}C$ isotopic ratio was then calculated on the delta per thousand scale by comparing the results with the one of the working reference (Glutamic acid) which is calibrated versus the international standard beforehand.

$^{18}O$

The measurement of the $^{18}O/^{16}O$ isotopic ratio was done in a continuous flux of an organic compound. For this purpose the samples were burned by pyrolysis at the level of a microanalyzer oven and the carbon monoxide produced was sent into the mass spectrometer (OPTIMA coupled with an elemental analyzer Fisons NA1500 2 series, or, a mass spectrometer Delta V coupled to an elemental analyzer TC/EA from Thermoelectron). Different isotopes of 28, 29, 30 were determined in the carbon monoxide produced by pyrolysis. The $^{18}O/^{16}O$ isotopic ratio was then calculated on the delta per thousand scale by comparing the results with SMOW.

Results

For PDO produced from raw glycerol we obtained an average $\delta^{13}C$ value of -35.57 $\pm$ 0.52 ‰, PDO produced from glucose has an average $\delta^{13}C$ value of -12.6 ‰ and PDO produced chemically has an average $\delta^{13}C$ value of - 30.05 $\pm$ 5.02 ‰.

PDO produced from raw glycerol has an average $\delta^{18}O$ value of 19.76 $\pm$ 2.42 ‰, PDO produced from glucose has an average $\delta^{18}O$ value of 22.0 ‰ and PDO produced chemically has an average $\delta^{18}O$ value of - 0.80 $\pm$ 1.27 ‰.

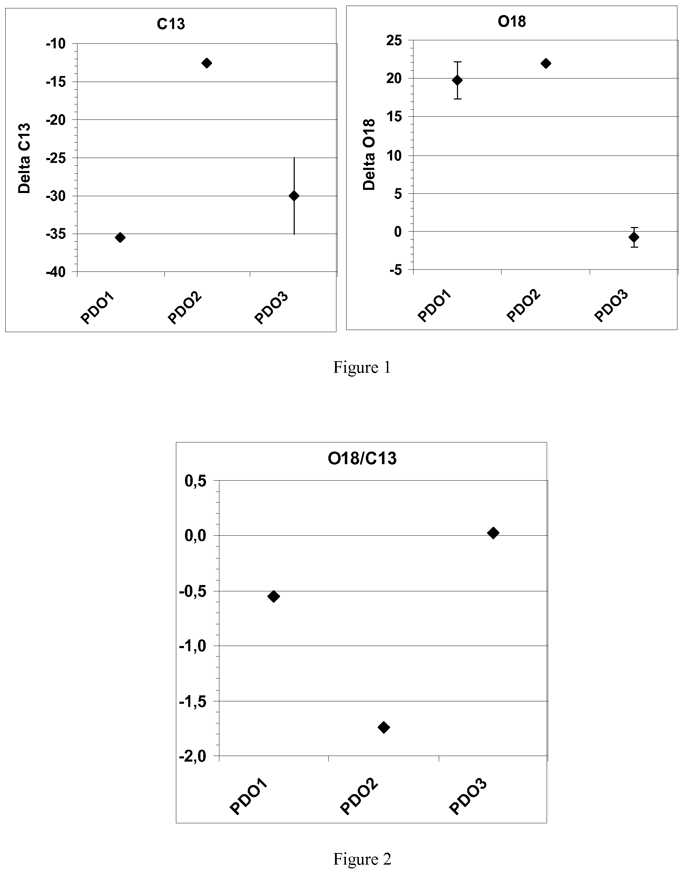

Thus the measurement of $\delta^{13}C$ allows distinguishing PDO produced by a fermentation process from diverse raw material. The $\delta^{13}C$ of chemically produced PDO shows high variability (-30.05 $\pm$ 5.02) and is only slightly higher than the $\delta^{13}C$ of PDO produced from glycerol. The $\delta^{18}O$ of the chemically produced PDO has a very low value (-0.8 $\pm$ 1.27) which makes it possible to distinguish between a chemically produced PDO and a PDO from biological origin, either glucose or glycerol based (Fig 1).

The $\delta^{18}O$ / $\delta^{13}C$ value clearly allows identifying the different PDOs (Fig 2) with a $\delta^{18}O$ / $\delta^{13}C$ ratio of -0.56 for glycerol based PDO, -1.75 for glucose based PDO and 0.03 for chemically produced PDO. In conclusion, PDO produced from glycerol can be identified via the measurements of $\delta^{13}C$ and $\delta^{18}O$ followed by the determination of the $\delta^{18}O$ / $\delta^{13}C$.

References

1. Cotte JF, Casabianca H, Lhéritier J, Perrucchietti C, Sanglar C, Waton H and Grenier-Loustalot MF. 2007. Study and validity of 13C stable carbon isotopic ratio analysis by mass spectrometry and 2H site specific natural isotopic fractionation by nuclear magnetic resonance isotopic measurements to characterize and control the authenticity of honey. Analytica Chimica Acta 582: 125-136.

2. Gonzalez-Pajuelo M, Meynial-Salles I, Mendes F, Andrade JC, Vasconcelos I, and Soucaille P. 2005. Metabolic engineering of Clostridium acetobutylicum for the industrial production of 1,3-propanediol from glycerol. Metabolic Engineering 7: 329-336.

3. Gonzalez-Pajuelo M, Meynial-Salles I, Mendes F, Soucaille P. and Vasconcelos I. 2006. Microbial conversion of a natural producer, Clostridium butyricum VPI 3266, and an engineered strain, Clostridium acetobutylicum DG (pSPD5). Applied and Environmental Microbiology, 72: 96-101.

4. Guillou C, Jamin E, Martin GJ, Reniero F, Wittkowski R and Wood R. 2001. Analyses isotopiques du vin et des produits derives du raisin. Bulletin de l'O.I.V : 839-840.

5. Nakas JP, Schaedle M, Parkinson CM, Coonley CE, and Tanenbaum SW. 1983. System development for linked-fermentation products of solvents from algal biomass. Applied and Environmental Microbiology 46: 1017-1023.

6. Papanikolaou S, Ruiz-Sanchez P, Pariset B, Blanchard F and Fick M. 2000. High production of 1,3-propanediol from industrial glycerol by a newly isolated Clostridium butyricum strain. Journal of Biotechnology. 77: 191-2008.

7. WO2006/128381. Method for preparing 1,3-propanediol by using glycerine as the by-product of the biological diesel oil.

8. WO01/11070. 1,3-propanediol and polymer derivatives from a fermentable carbon source.

**Claims**

1. A method for the production of 1,3-propanediol in a continuous fermentation process, comprising culturing a strain of *Clostridium* genetically modified to have its glycerol metabolism directed to 1,3-propanediol production on a culture medium, and recovering the 1,3-propanediol, **characterized in that**

   - the feed medium comprises a high concentration of industrial glycerine, said industrial glycerine being a by-product from biodiesel production and comprising of more than 70% of glycerol, said glycerol being present at a concentration comprised between 90 and 120g/l, and the dilution rate is comprised between 0.005 and $0.1h^{-1}$
   - wherein the strain of *Clostridium* is previously adapted by a continuous culture at a dilution rate comprised between 0.005 and $0.1h^{-1}$ in the presence of a high concentration of industrial glycerine in the feed medium, said industrial glycerine comprising of more than 70% of glycerol, said glycerol being present at a concentration comprised between 90 and 120g/l
   - wherein said culture medium does not contain any organic nitrogen source.

2. The method according to claim 1 **characterized in that** the strain of *Clostridium* is a strain of *Clostridium acetobutylicum.*

3. The method according to any one of claims 1 to 2, **characterized in that** the glycerol dehydratase activity in the genetically modified strain of *Clostridium* is independent of the presence of coenzyme B12 or one of its precursors and is derived from *Clostridium butyricum.*

4. The method according to any one of claims 1 to 3, **characterized in that** the genetically modified strain of *Clostridium* is adapted into a producing microorganism by culturing the microorganism on a culture medium comprising high concentration of industrial glycerine at a low dilution rate comprised between 0.005 and $0.1h^{-1}$ and selecting the adapted microorganism able to grow on the culture medium having high concentration of industrial glycerine.

5. The method according to any one of claims 1 to 4, **characterized in that** the 1,3-propanediol is further purified.

6. A method for the modification of a microorganism of the genus *Clostridium* into a microorganism adapted to grow in the presence of a high concentration of industrial glycerine, said industrial glycerine being a by-product from biodiesel production and comprising more than 70% of glycerol, said method comprising continuously culturing the microorganism on a culture medium not containing any organic nitrogen source, the feed medium comprising a high concentration of industrial glycerine, the glycerol being present at a concentration comprised between 90 and 120g/l, and being fed at a low dilution rate comprised between 0.005 and $0.1h^{-1}$ and over a period ranging from 24 hours to 10 days, and selecting the adapted microorganism able to grow on the culture medium having high concentration of industrial glycerine.

7. A microorganism of the genus *Clostridium* genetically modified to have its glycerol metabolism directed to 1,3-propanediol production adapted to grow in the presence of a high concentration of industrial glycerine and in a culture medium which does not contain any organic nitrogen source susceptible to be obtained by the method as claimed in claim 6, said industrial glycerine being a by-product from biodiesel production and comprising more than 70% of glycerol, and in the feed medium the glycerol of a continuous culture being present at a concentration comprised between 90 and 120g/l.

**Patentansprüche**

1. Verfahren zur Herstellung von 1,3-Propandiol in einem kontinuierlicher Fermentierungsprozess, das das Kultivieren eines Stamms von Clostridium umfasst, der genetisch modifizierte wurde, sodass sein Glycerol-Stoffwechsel auf die Erzeugung von 1,3-Propandiol in einem Kulturmedium gerichtet ist, und Gewinnen des 1,3 Butandiol, **dadurch gekennzeichnet, dass**

   - das Nährmedium eine hohe Konzentration von industriellem Glycerin enthält, wobei das industrielle Glycerin ein Nebenprodukt der Biodiesel-Herstellung ist und mehr als 70 % Glycerol enthält, wobei das Glycerol in einer Konzentration vorhanden ist, die zwischen 90 und 120 g/l liegt, und die Verdünnungsrate liegt zwischen 0,005 und 0,1 h-1,
   - wobei der Stamm von Clostridium vorher angepasst wurde durch eine kontinuierliche Kultur bei einer Verdünnungsrate, die zwischen 0,005 und 0,1 h-1 liegt, in Gegenwart einer hohen Konzentration von industriellem Glycerin im Nährmedium, wobei das industrielle Glycerin mehr als 70 % Glycerol enthält, wobei das Glycerol in einer Konzentration zwischen 90 und 120 g/l vorliegt,
   - wobei das Nährmedium keine organische Stickstoffquelle enthält.

2. Verfahren nach Anspruch 1, das **dadurch gekennzeichnet ist, dass** der Stamm von Clostridium ein Stamm von Clostridium acetobutylicum ist.

3. Verfahren nach einem der Ansprüche 1 bis 2, **dadurch gekennzeichnet, dass** die Glycerol-Dehydratase-Aktivität im genetisch modifizierten Stamm von Clostridium unabhängig vom Vorhandensein von Coenzym B12 oder einem seiner Vorläufer ist und aus Clostridium butyricum abgeleitet wurde.

4. Verfahren nach einem der Ansprüche 1 bis 3, **dadurch gekennzeichnet, dass** der genetisch modifizierte Stamm von Clostridium an einen produzierenden Mikroorganismus angepasst wurde, in dem der Mikroorganismus auf einem Nährmedium kultiviert wurde, das eine hohe Konzentration von industriellem Glycerin bei einer niedrigen Verdünnungsrate aufweist, die zwischen 0,005 und 0,1 h-1 liegt, und durch Auswählen der angepassten Mikroorganismen, die in der Lage sind, auf dem Nährmedium zu wachsen, das eine hohe Konzentration von industriellem Glycerin enthält.

5. Verfahren nach einem der Ansprüche 1 bis 4, **dadurch gekennzeichnet, dass** das 1,3-Propandiol weitergehend gereinigt wird.

6. Verfahren zur Modifizierung eines Mikroorganismus der Gattung Clostridium zu einem Mikroorganismus, der dafür angepasst ist, in Gegenwart einer hohen Konzentration von industriellem Glycerin zu wachsen,
   wobei das industrielle Glycerin ein Nebenprodukt der Biodiesel-Herstellung ist, und
   mehr als 70 % Glycerol enthält,
   wobei das Verfahren das kontinuierliche Züchten des Mikroorganismus auf einem Kulturmedium umfasst, das keine organische Stickstoffquelle enthält, wobei das Nährmedium eine hohe Konzentration von industriellem Glycerin enthält, wobei das Glycerol in einer Konzentration vorhanden ist, die zwischen 90 und 120 g/l beträgt, und bei einer niedrigen Verdünnungsrate zugeführt wird, die zwischen 0,005 und 0,1 h-1 liegt und über einen Zeitraum, der von 24 Stunden bis zu 10 Tagen reicht, und
   Auswählen des angepassten Mikroorganismus, der in der Lage ist, auf dem Nährmedium zu wachsen, welches eine hohe Konzentration von industriellem Glycerin hat.

7. Mikroorganismus der Gattung Clostridium, der genetisch modifiziert ist, sodass sein Glycerol-Stoffwechsel auf die Erzeugung von 1,3-Propandiol ausgerichtet ist, der dafür angepasst ist, in Gegenwart einer hohen Konzentration von industriellem Glycerin zu wachsen und in einem Nährmedium, das keine organische Stickstoffquelle hat, das zugänglich dafür ist, durch das Verfahren nach Anspruch 6 gewonnen zu werden, wobei das industrielle Glycerin ein Nebenprodukt aus der Biodiesel-Herstellung ist und mehr als 70 % Glycerol enthält, und wobei in dem Nährmedium das Glycerol einer kontinuierlichen Kultur in einer Konzentration vorhanden ist, die zwischen 90 und 120 g/l beträgt

**Revendications**

1. Procédé pour la production de 1,3-propanediol dans un processus de fermentation en continu, comprenant la culture d'une souche de *Clostridium* génétiquement modifiée pour que son métabolisme du glycérol soit dirigé vers la production de 1,3-propanediol sur un milieu de culture, et la récupération du 1,3-propanediol, **caractérisé en ce que**

- le milieu d'alimentation comprend une forte concentration de glycérine industrielle, ladite glycérine industrielle étant un sous-produit de la production de biodiésel et comprenant plus de 70 % de glycérol, ledit glycérol étant présent à une concentration comprise entre 90 et 120 g/l, et le taux de dilution est compris entre 0,005 et 0,1 h$^{-1}$,
- dans lequel la souche de *Clostridium* a été antérieurement adaptée par une culture continue à un taux de dilution compris entre 0,005 et 0,1 h$^{-1}$ en présence d'une forte concentration de glycérine industrielle dans le milieu d'alimentation, ladite glycérine industrielle comprenant plus de 70 % de glycérol, ledit glycérol étant présent à une concentration comprise entre 90 et 120 g/l,
- dans lequel ledit milieu de culture ne contient aucune source d'azote organique.

2. Procédé selon la revendication 1, **caractérisé en ce que** la souche de *Clostridium* est une souche de *Clostridium acetobutylicum.*

3. Procédé selon l'une quelconque des revendications 1 et 2, **caractérisé en ce que** l'activité de glycérol déshydratase dans la souche génétiquement modifiée de *Clostridium* est indépendante de la présence de coenzyme B12 ou de l'un de ses précurseurs et est dérivée de *Clostridium butyricum.*

4. Procédé selon l'une quelconque des revendications 1 à 3, **caractérisé en ce que** la souche génétiquement modifiée de *Clostridium* est adaptée en un microorganisme producteur par culture du microorganisme sur un milieu de culture comprenant une forte concentration de glycérine industrielle à un faible taux de dilution compris entre 0,005 et 0,1 h$^{-1}$ et sélection du microorganisme adapté capable de croître sur le milieu de culture ayant une forte concentration de glycérine industrielle.

5. Procédé selon l'une quelconque des revendications 1 à 4, **caractérisé en ce que** le 1,3-propanediol est en outre purifié.

6. Procédé pour la modification d'un microorganisme du genre *Clostridium* en un microorganisme adapté pour croître en présence d'une forte concentration de glycérine industrielle, ladite glycérine industrielle étant un sous-produit de la production de biodiésel et comprenant plus de 70 % de glycérol, ledit procédé comprenant la culture en continu du microorganisme sur un milieu de culture ne contenant aucune source d'azote organique, le milieu d'alimentation comprenant une forte concentration de glycérine industrielle, le glycérol étant présent à une concentration comprise entre 90 et 120 g/l, et étant introduit à un faible taux de dilution compris entre 0,005 et 0,1 h$^{-1}$ et sur une période allant de 24 heures à 10 jours, et la sélection du microorganisme adapté capable de croître sur le milieu de culture ayant une forte concentration de glycérine industrielle.

7. Microorganisme du genre *Clostridium* génétiquement modifié pour que son métabolisme du glycérol soit dirigé vers la production de 1,3-propanediol, adapté pour croître en présence d'une forte concentration de glycérine industrielle et dans un milieu de culture qui ne contient aucune source d'azote organique, susceptible d'être obtenu par le procédé selon la revendication 6, ladite glycérine industrielle étant un sous-produit de la production de biodiésel et comprenant plus de 70 % de glycérol, et le glycérol étant présent dans le milieu d'alimentation d'une culture continue à une concentration comprise entre 90 et 120 g/l.

Figure 1

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006128381 A
- WO 0111070 A

### Non-patent literature cited in the description

- **COTTE JF ; CASABIANCA H ; LHÉRITIER J ; PER-RUCCHIETTI C ; SANGLAR C ; WATON H ; GRENIER-LOUSTALOT MF.** Study and validity of 13C stable carbon isotopic ratio analysis by mass spectrometry and 2H site specific natural isotopic fractionation by nuclear magnetic resonance isotopic measurements to characterize and control the authenticity of honey. *Analytica Chimica Acta,* 2007, vol. 582, 125-136
- **GONZALEZ-PAJUELO M ; MEYNIAL-SALLES I ; MENDES F ; ANDRADE JC ; VASCONCELOS I ; SOUCAILLE P.** Metabolic engineering of Clostridium acetobutylicum for the industrial production of 1,3-propanediol from glycerol. *Metabolic Engineering,* 2005, vol. 7, 329-336
- **GONZALEZ-PAJUELO M ; MEYNIAL-SALLES I ; MENDES F ; SOUCAILLE P. ; VASCONCELOS I.** Microbial conversion of a natural producer, Clostridium butyricum VPI 3266, and an engineered strain, Clostridium acetobutylicum DG (pSPD5). *Applied and Environmental Microbiology,* 2006, vol. 72, 96-101
- **GUILLOU C ; JAMIN E ; MARTIN GJ ; RENIERO F ; WITTKOWSKI R ; WOOD R.** Analyses isotopiques du vin et des produits derives du raisin. *Bulletin de l'O.I.V,* 2001, 839-840
- **NAKAS JP ; SCHAEDLE M ; PARKINSON CM ; COONLEY CE ; TANENBAUM SW.** System development for linked-fermentation products of solvents from algal biomass. *Applied and Environmental Microbiology,* 1983, vol. 46, 1017-1023
- **PAPANIKOLAOU S ; RUIZ-SANCHEZ P ; PARISET B ; BLANCHARD F ; FICK M.** High production of 1,3-propanediol from industrial glycerol by a newly isolated Clostridium butyricum strain. *Journal of Biotechnology,* 2000, vol. 77, 191-2008